(19) Europäisches Patentamt

European Patent Office

Office européen des brevets

(11) EP 0 757 043 A1

(12) EUROPÄISCHE PATENTANMELDUNG

(43) Veröffentlichungstag:
05.02.1997 Patentblatt 1997/06

(51) Int. Cl.⁶: C07D 301/12

(21) Anmeldenummer: 96108370.6

(22) Anmeldetag: 25.05.1996

(84) Benannte Vertragsstaaten:
AT BE DE DK ES FI FR GB IT NL PT SE

(30) Priorität: 01.08.1995 DE 19528219

(71) Anmelder: Degussa Aktiengesellschaft
60311 Frankfurt (DE)

(72) Erfinder: Thiele, Georg, Dr.
63452 Hanau (DE)

(54) **Verfahren zur Herstellung von Epoxiden aus Olefinen**

(57) Verfahren zur Herstellung von Epoxiden aus Olefinen und Wasserstoffperoxid in Gegenwart eines Titanatome enthaltenden Zeolithen als Katalysator, dem vor oder während der Reaktion neutral oder sauer reagierende Salze zugesetzt werden.

EP 0 757 043 A1

**Beschreibung**

Die Erfindung betrifft ein Verfahren zur Herstellung von Epoxiden aus Olefinen.

Es ist bekannt, Epoxide durch Umsetzung von Olefinen mit Wasserstoffperoxid in Gegenwart von Titansilicalit TS1 herzustellen (EP-A 0 100 119). Das bekannte Verfahren hat den Nachteil, daß die Epoxidausbeute durch säurekatalysierte Folgereaktionen des Epoxides mit Wasser oder dem in der Reaktionsmischung vorhandenen Lösungsmittel, die durch den schwach sauren Titansilicalit TS1 ausgelöst werden, herabgesetzt wird.

Es ist weiterhin bekannt, Epoxide durch Umsetzung von Olefinen mit Wasserstoffperoxid in Gegenwart eines mit Basen vorbehandelten Titansilicalit TS1 herzustellen (EP-A 0 230 949) . Bei diesem Verfahren muß die zur Neutralisation des Katalysators (Titansilicalit TS1) verwendete Basenmengen in engen Grenzen gewählt werden, weil ein Überschreiten der Grenze zu einem starken Aktivitätsverlust des Katalysators führt (M. G. Clerici et al., Journal of Catalysis 140, 71 - 83 (1993). Das bekannte Verfahren hat den Nachteil, daß die Behandlung mit größeren Basenmengen zu einem starken Aktivitätsverlust bis hin zur vollständigen Hemmung der Reaktion führt.

Gegenstand der Erfindung ist ein Verfahren zur Herstellung von Epoxiden aus Olefinen und Wasserstoffperoxid in Gegenwart eines Titanatome enthaltenden Zeolithen als Katalysator, welches dadurch gekennzeichnet ist, daß man dem Katalysator vor oder während der Reaktion neutral oder sauer reagierende Salze zusetzt.

Das neutral oder sauer reagierende Salz kann ein oder mehrere Kationen aus der Gruppe $Li^+$, $Na^+$, $K^+$, $NH_4^+$, $RNH_3^+$ oder $R_2NH_2^+$ enthalten, wobei R für einen Alkylrest mit 1 bis 6 Kohlenstoffatomen steht.

Als neutral- oder sauer reagierendes Salz kann vorzugsweise ein Salz der Zusammensetzung $M^+X^-$ oder der Zusammensetzung $M_2SO_4$ verwendet werden, wobei $M^+$ für $Li^+$, $Na^+$, $K^+$, oder $NH_4^+$ und $X^-$ für $NO_3^-$, $ClO_4^-$, $Cl^-$ oder $H_2PO_4^-$ steht.

In einer Ausführung der Erfindung wird der Titanatome enthaltende Katalysator vor seiner Verwendung zur Epoxidierung mit einer Lösung eines neutral oder sauer reagierenden Salzes in Wasser oder einem Alkohol umgesetzt, von dieser Lösung abgetrennt, gegebenenfalls mit Wasser und/oder einem Lösungsmittel gewaschen und anschließend zur Epoxidierung eines Olefins eingesetzt. Die Konzentration der zur Behandlung des Katalysators verwendeten Salzlösung kann zwischen 0,0001 mol/l und der Sättigungsgrenze, vorzugsweise zwischen 0,01 und 1 mol/l, variiert werden, wobei im Gegensatz zum Stand der Technik auch Konzentrationen von 0,1 mol/l und höher keine nachteilige Wirkungen auf die katalytische Aktivität des behandelten Katalysators in der Epoxidierungsreaktion haben. Temperatur und Dauer der Katalysatorbehandlung können in weiten Grenzen gewählt werden und werden nur begrenzt durch die Geschwindigkeit des Stoffaustausches zwischen Katalysator und Salzlösung bei niedriger Temperatur und kurzer Behandlungszeit und die Stabilität des Katalysators gegenüber Hydrolyse bei hohen Temperaturen und langen Behandlungszeiten. Die zur Behandlung des Katalysators verwendete Salzlösung kann vorteilhaft mehrfach wieder eingesetzt werden, wobei die Zahl der möglichen Wiederverwendungen von der Konzentration der Salzlösung und dem Mengenverhältnis von Salzlösung und behandeltem Katalysator abhängt.

In einer anderen Ausführungsform der Erfindung erfolgt die Behandlung des Katalysators während der Epoxidierungsreaktion. Dazu wird das Salz, gegebenenfalls als Lösung in Wasser oder einem anderen Lösungsmittel, entweder direkt der Reaktionsmischung der Epoxidierung oder einem der Einsatzstoffe der Epoxidierungsreaktion zugesetzt. Die Salzmenge kann dabei so gewählt werden, daß die Salzkonzentration in der Reaktionsmischung zwischen 0,0001 und 1 mol/l, vorzugsweise zwischen 0,001 und 1 mol/l, liegt. Das neutral oder sauer reagierende Salz kann dabei vorteilhaft dem zur Epoxidierung eingesetzten Wasserstoffperoxid zugesetzt werden, ohne sich nachteilig auf dessen Lagerstabilität auszuwirken.

Bei einer kontinuierlichen Durchführung der Epoxidierungsreaktion kann die erfindungsgemäße Behandlung des Katalysators sowohl durch eine kontinuierliche als auch durch eine intermittierende Zugabe des zur Katalysatorbehandlung verwendeten Salzes erfolgen.

Die Epoxidierung von Olefinen mit Wasserstoffperoxid in Gegenwart eines Titanatome enthaltenden Katalysators, der vor oder während der Epoxidierungsreaktion mit einem neutralen oder sauren wasserlöslichen Salz behandelt wird, besitzt gegenüber bekannten Verfahren den Vorteil, daß bei verringerter Nebenproduktbildung eine Variation der Menge des zur Behandlung des Katalysators eingesetzten Stoffs sich nicht nachteilig auf die katalytische Aktivität des Katalysators bei der Epoxidierungsreaktion auswirkt.

Das erfindungsgemäße Verfahren kann auf alle Olefine angewandt werden, die auch mit dem nicht vorbehandelten Katalysator epoxidiert werden können. Beispielsweise können die Olefine, die in dem Dokument EP-A 0 100 119 aufgeführt werden, eingesetzt werden. Bevorzugt können unverzweigte oder mit Methylgruppen substituierte Olefine mit 2 bis 16 Kohlenstoffatomen sowie Allylchlorid und Allylalkohol, insbesondere Propylen, 1-Buten, 2-Buten, Isobuten oder 1-Penten eingesetzt werden.

Als Titanatome enthaltende Zeolithe können folgende bekannte Verbindungen eingesetzt werden: Titansilicalit-1 mit MFI-Struktur, Titansilicalit-2 mit MEL-Struktur, Titan-Beta-Zeolith, TS-48 mit der Struktur von Zeolith ZSM-48 sowie Titan-Mordenit mit MOR-Struktur. Diese Zeolithe sind aus folgender Literatur bekannt:

Titansilicalit-1 (TS1)     DE 30 47 798

| Titansilicalit-2 (TS2) | BE 1 001 038; J.S. Reddy, R. Kumar, P.Ratnasamy, Appl. Catal. 58 (1990) L1 |
| Titan-Beta-Zeolith (Ti-Beta) | ES 2 037 596, M.A. Camblor, A. Corma, A. Martinez, J. Perez-Pariente, J. Chem. Soc., Chem. Commun. 1992, 589 |
| TS-48 | D.P. Serrano, H.X.Li, M.E. Davis, J. Chem. Soc., Chem. Commun. 1992, 745 |
| Titan-Mordenit | G.J. Kim, B.R.Cho, J.H. Kim, Catal. Letters 1993, 259 |

Bevorzugt werden Titansilicalit-1 und Titansilicalit-2 eingesetzt, besonders bevorzugt Titansilicalit-1.

**Beispiele**

Für die folgenden Beispiele werden drei Proben von Titansilicalit verwendet, die nach den Angaben in J.P.A. Martens et al., Appl. Catal. A $\underline{99}$ (1993) 71 - 84 hergestellt werden und im folgenden als Katalysator 1, 2 und 3 bezeichnet werden.

**Beispiel 1**

*Bestimmung der katalytischen Aktivität von Titansilicalit in der Epoxidierung von Propylen mit Wasserstoffperoxid*

In einem thermostatisierten Laborautoklav mit Begasungsrührer werden 1 g Katalysator 1 in 300 ml Methanol bei 40°C unter Propylenatmosphäre vorgelegt und bei 3 bar Überdruck mit Propylen gesättigt. Dann werden unter Rühren 13,1 g 30 Gew.-% wäßrige Wasserstoffperoxidlösung in einer Portion zugegeben, und die Reaktionsmischung bei 40°C und 3 bar gehalten, wobei über einen Druckregler Propylen nachdosiert wird, um den Verbrauch durch die Reaktion auszugleichen. In regelmäßigen Abständen werden über ein Filter Proben entnommen, und der Wasserstoffperoxidgehalt der Reaktionsmischung durch Redoxtitration mit Cer(IV)sulfat-Lösung bestimmt. Die Auftragung von ln $(c/c_0)$ gegen die Zeit t, wobei c die gemessene $H_2O_2$-Konzentration zum Zeitpunkt t und $C_0$ die berechnete $H_2O_2$-Konzentration zu Beginn der Reaktion ist, ergibt eine Gerade. Aus der

$$\frac{dc}{dt} = -k.c.c_{kat},$$

Steigung der Geraden wird mit der Beziehung worin $c_{kat}$ für die Katalysatorkonzentration in kg Katalysator je kg Reaktionsmischung steht, die Aktivitätskennzahl k zu 15,0 $min^{-1}$ bestimmt.

**Beispiele 2 - 4**

*Katalytische Aktivität von erfindungsgemäß behandeltem Titansilicalit in der Epoxidierung von Propylen*

5 g Katalysator 1 werden 4 h bei 20°C in 500 ml einer Lösung von 0,1 mol/l eines neutralen bzw. sauren Salzes in vollentsalztem Wasser suspendiert, anschließend filtriert, mit vollentsalztem Wasser und Methanol gewaschen und bei 20°C an der Luft getrocknet. Von den derart behandelten Katalysatorproben wird die katalytische Aktivität in der Epoxidierung von Propylen gemäß Beispiel 1 bestimmt. Tabelle 1 zeigt die zur Behandlung des Katalysators eingesetzten Salze und die katalytischen Aktivitäten der behandelten Katalysatorproben.

Tabelle 1

| Katalytische Aktivität von Titansilicalit in der Epoxidierung von Propylen nach Behandlung mit neutralen bzw. sauren Salzen | | |
|---|---|---|
| **Beispiel** | **Zur Behandlung eingesetztes Salz** | **Aktivitätskennzahl in $min^{-1}$** |
| 1 | -- | 15,0 |
| 2 | $Na_2SO_4$ | 16,4 |
| 3 | $(NH_4)_2SO_4$ | 11,7 |
| 4 | $NH_4NO_3$ | 9,4 |

**Vergleichsbeispiele 5 - 8**

*Katalytische Aktivität von nach dem Stand der Technik mit Basen behandeltem Titansilicalit in der Epoxidierung von Propylen*

Die Beispiele 2 - 4 werden wiederholt, aber anstelle des neutralen bzw. sauren Salzes wird eine 0,1 mol/l einer Base enthaltende Lösung in vollentsalztem Wasser zur Behandlung des Katalysators eingesetzt. Tabelle 2 zeigt die zur Behandlung des Katalysators eingesetzten Basen und die katalytische Aktivität der behandelten Katalysatorproben:

Tabelle 2

| Katalytische Aktivität von Titansilicalit in der Epoxidierung von Propylen nach Behandlung mit Basen | | |
|---|---|---|
| **Beispiel** | **Zur Behandlung eingesetzte Base** | **Aktivitätskennzahl in $min^{-1}$** |
| 1 | -- | 15,0 |
| 5 | NaOAc | 2,2 |
| 6 | $NH_4OAc$ | 1,1 |
| 7 | $NH_3$ | 0,3 |
| 8 | NaOH | 0,1 |

Die Beispiele 2 - 4 und die Vergleichsbeispiele 5 - 8 zeigen, daß sich die erfindungsgemäße Behandlung des Katalysators nur wenig auf die Aktivität des Katalysators in der Epoxidierung von Propylen auswirkt, während die Behandlung des Katalysators mit Basen gemäß dem Stand der Technik unter sonst gleichen Bedingungen einen starken Verlust an katalytischer Aktivität bewirkt.

**Beispiel 9**

*Bestimmung der Nebenproduktbildung bei der Epoxidierung von Propylen mit Wasserstoffperoxid und einem Titansilicalit-Katalysator*

Beispiel 1 wird mit Katalysator 2 wiederholt. Die Aktivitätskennzahl wird mit 23,7 $min^{-1}$ bestimmt. Zusätzlich wird 2 h nach Zugabe des Wasserstoffperoxids eine Probe entnommen und gaschromatographisch der Gehalt des Hauptprodukts Propylenoxid und der Nebenprodukte 1-Methoxy-2-propanol, 2-Methoxy-1-propanol und 1,2-Propandiol ermittelt. Der Anteil der drei Nebenprodukte an der Gesamtmenge der Produkte wird zu 8,6 mol-% bestimmt.

**Beispiele 10 - 12**

*Nebenproduktbildung in der Epoxidierung von Propylen mit erfindungsgemäß behandeltem Titansilicalit*

Die Beispiele 2 - 4 werden mit Katalysator 2 wiederholt. Die katalytische Aktivität der behandelten Katalysatorproben und der Anteil der Nebenproduktbildung wird wie in Beispiel 9 ermittelt. Tabelle 3 zeigt die Ergebnisse der Beispiele 10 - 12.

**Vergleichsbeispiel 13**

*Nebenproduktbildung mit nach dem Stand der Technik behandelten Titansilicalit in der Epoxidierung von Propylen*

Das Vergleichsbeispiel 5 wird mit Katalysator 2 wiederholt. Die katalytische Aktivität der neutralisierten Katalysatorprobe und der Anteil der Nebenprodukte wird wie in Beispiel 9 ermittelt und ist in Tabelle 3 aufgeführt.

Tabelle 3

| Katalytische Aktivität und Anteil der Nebenprodukte an der Gesamtmenge der Produkte bei der Epoxidierung von Propylen mit vorbehandeltem Titansilicalit | | | |
|---|---|---|---|
| Beispiel | Zur Behandlung eingesetzter Stoff | Aktivitätskennzahl in min$^{-1}$ | Anteil der Nebenprodukte in mol-% |
| 9 | -- | 23,7 | 8,6 |
| 10 | $Na_2SO_4$ | 21,4 | 2,6 |
| 11 | $(NH_4)_2SO_4$ | 15,8 | 1,6 |
| 12 | $NH_4NO_3$ | 20,6 | 2,3 |
| 13 | NaOAc | 4,5 | 0,7 |

Die Beispiele 10 - 12 zeigen im Vergleich mit Beispiel 9, daß die erfindungsgemäße Behandlung des Katalysators mit neutralen oder sauren Salzen zu einer wesentlichen Verringerung des Anteils der Nebenproduktbildung bei der Epoxidierung von Olefinen mit Wasserstoffperoxid führt.

**Beispiel 14**

Beispiel 1 wird mit Katalysator 3 wiederholt. Die Aktivitätskennzahl wird mit 21,2 min$^{-1}$ bestimmt.

**Beispiel 15**

*Katalytische Aktivität von mit $NaH_2PO_4$ behandeltem Titansilicalit in der Epoxidierung von Propylen*

Die Beispiele 2 - 4 werden mit Katalysator 3 wiederholt, als saures Salz wird jedoch $NaH_2PO_4$ eingesetzt. Die katalytische Aktivität wird wie in Beispiel 1 ermittelt und ist in Tabelle 4 aufgeführt.

**Beispiel 16 (Vergleichsbeispiel)**

*Katalytische Aktivität von mit $Na_2HPO_4$ neutralisiertem Titansilicalit in der Epoxidierung von Propylen*

Die Beispiele 5 - 8 werden mit Katalysator 3 wiederholt, als Base wird jedoch $Na_2HPO_4$ eingesetzt. Die katalytische Aktivität wird wie in Beispiel 1 ermittelt und ist in Tabelle 4 aufgeführt.

Tabelle 4

| Katalytische Aktivität von Titansilicalit in der Epoxidierung von Propylen nach Behandlung mit $NaH_2PO_4$ bzw. $Na_2HPO_4$ | | |
|---|---|---|
| Beispiel | Zur Behandlung eingesetzte Base | Axtivitätskennzahl in min$^{-1}$ |
| 14 | -- | 21,2 |
| 15 | $NaH_2PO_4$ | 12,7 |
| 16 | $Na_2HPO_4$ | 0,3 |

Das Beispiel 15 und das Vergleichsbeispiel 16 zeigen, daß sich die erfindungsgemäße Behandlung des Katalysators mit dem sauer reagierenden Salz $NaH_2PO_4$ nur wenig auf die Aktivität des Katalysators in der Epoxidierung von Propylen auswirkt. Im Vergleich dazu bewirkt die Behandlung des Katalysators mit dem basisch reagierenden Salz $Na_2HPO_4$ unter sonst gleichen Bedingungen einen starken Verlust an katalytischer Aktivität.

**Beispiel 17**

*Katalytische Aktivität von Titansilicalit in der kontinuierlichen Epoxidierung von Propylen*

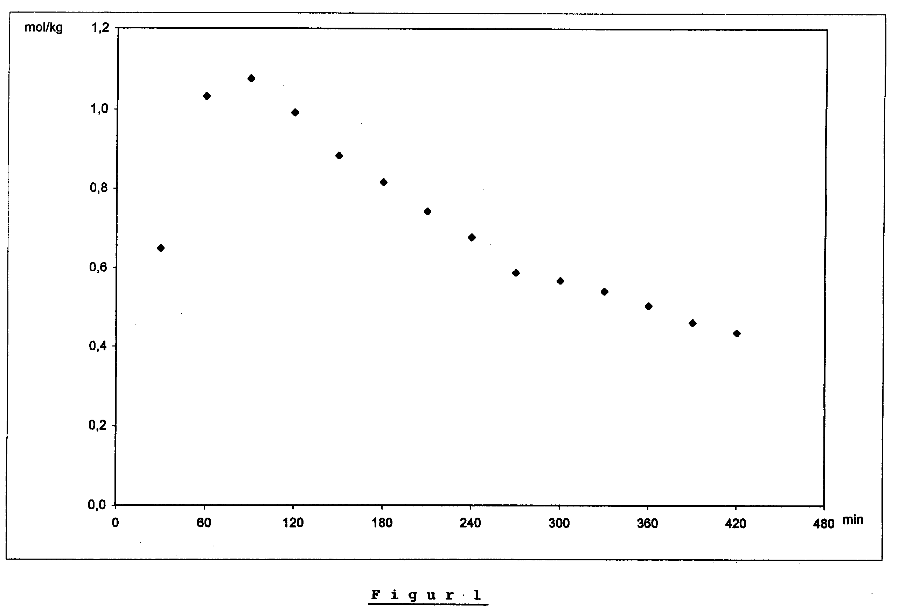

In einem thermostatisierten Laborautoklav mit Begasungsrührer werden 5 g Katalysator 3 in 295 g Methanol bei 40$^0$C unter Propylenatmosphäre vorgelegt und bei 3 bar Überdruck mit Propylen gesättigt. Dann wird unter Rühren eine Mischung aus 475 g 50 Gew.-% Wasserstoffperoxid, 2225 g Methanol und 560 g vollentsalztem Wasser mit einer Rate von 300 g/h zudosiert und gleichzeitig so viel Reaktionsmischung über ein Filter entnommen, daß das Gewicht des Reaktorinhalts konstant bleibt. Der Katalysator wird dabei durch das Filter im Reaktor zurückgehalten. Während der Wasserstoffperoxiddosierung wird über einen Druckregler Propylen nachdosiert, um den Druck im Reaktor konstant zu halten. Am Reaktorausgang werden in regelmäßigen Abständen Proben entnommen und der Gehalt an Reaktionsprodukten (Propylenoxid, 1-Methoxy-2-propanol und 2-Methoxy-1-propanol) gaschromatographisch bestimmt. Die Konzentration an Reaktionsprodukten am Reaktorausgang in mol/kg gegen die Zeit in min. ist in Figur 1 dargestellt.

**Beispiel 18**

*Katalytische Aktivität von erfindungsgemäß behandeltem Titansilicalit bei der kontinuierlichen Epoxidierung von Propylen*

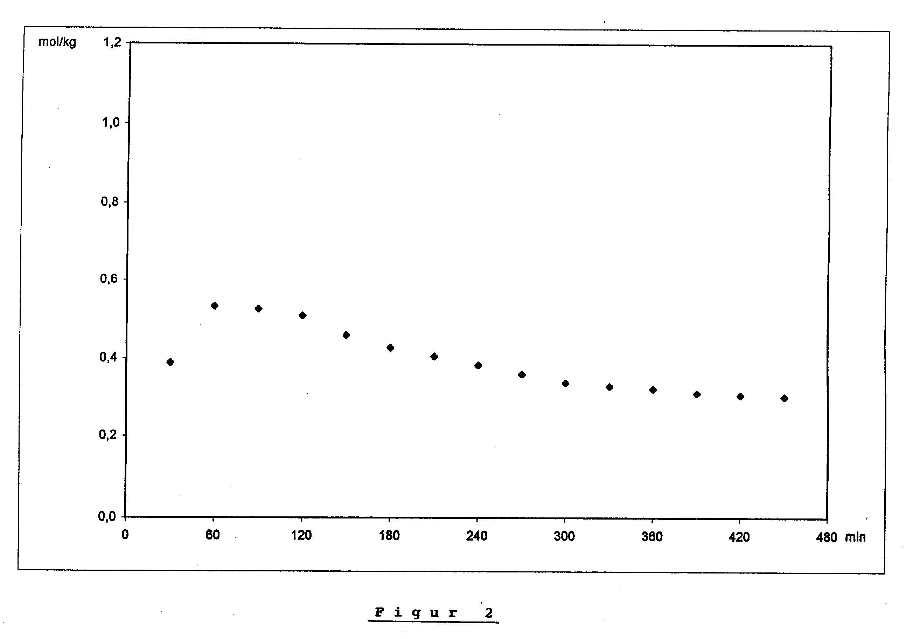

Beispiel 17 wird wiederholt. Der Mischung aus Methanol, Wasser und Wasserstoffperoxid wird jedoch zusätzlich 0.1 mol/kg Ammoniumnitrat zugesetzt. Die Konzentration an Reaktionsprodukten am Reaktorausgang in mol/kg gegen die Zeit in min. wird in Figur 2 dargestellt.

**Vergleichsbeispiel 19**

*Katalytische Aktivität von nach dem Stand der Technik neutralisiertem Titansilicalit in der kontinuierlichen Epoxidierung von Propylen*

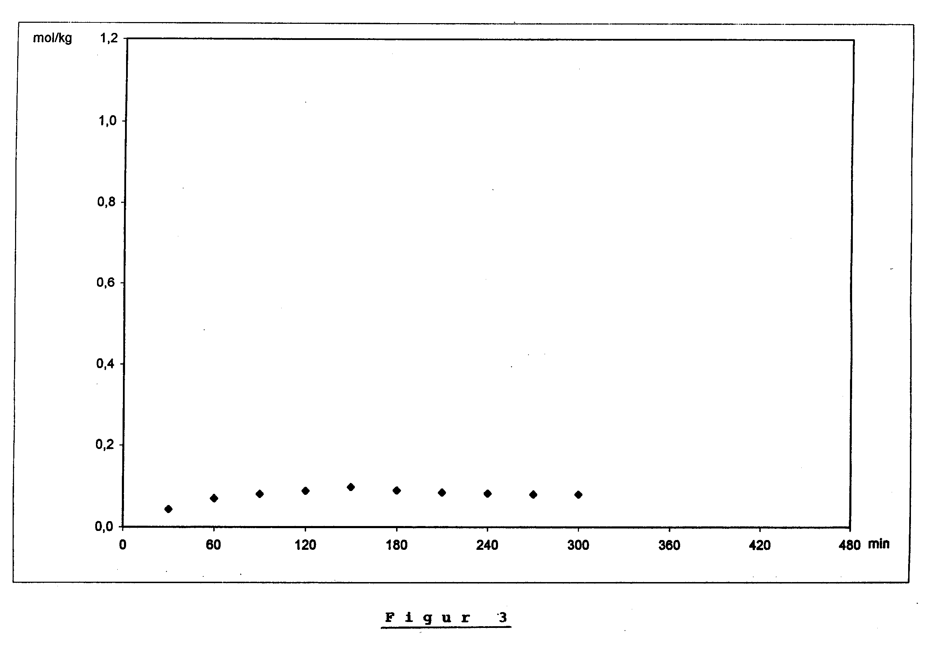

Beispiel 17 wird wiederholt. Der Mischung aus Methanol, Wasser und Wasserstoffperoxid wird jedoch zusätzlich 0.1 mol/kg Lithiumacetat zugesetzt. Die Konzentration an Reaktionsprodukten am Reaktorausgang in mol/kg gegen die Zeit in min. wird in Figur 3 dargestellt.

Die Beispiele 17 und 18 und das Vergleichsbeispiele 19 zeigen, daß sich unter sonst gleichen Reaktionsbedingungen die erfindungsgemäße Behandlung des Katalysators weniger nachteilig auf die Aktivität des Katalysators in der kontinuierlichen Epoxidierung von Propylen, als die Behandlung des Katalysators mit Basen nach dem Stand der Technik, auswirkt.

**Patentansprüche**

1. Verfahren zur Herstellung von Epoxiden aus Olefinen und Wasserstoffperoxid in Gegenwart eines Titanatome enthaltenden Zeolithen als Katalysator, dadurch gekennzeichnet, daß man dem Katalysator vor oder während der Reaktion neutral oder sauer reagierende Salze zusetzt.

2. Verfahren nach Anspruch 1, dadurch gekennzeichnet, daß das zur Behandlung des Katalysators verwendete Salz ein oder mehrere Kationen aus der Gruppe Li$^+$, Na$^+$, K$^+$, NH$_4^+$, RNH$_3^+$ oder R$_2$NH$_2^+$ enthält, wobei R für einen n-Alkylrest mit 1 bis 6 Kohlenstoffatomen steht.

3. Verfahren nach Anspruch 1 oder 2, dadurch gekennzeichnet, daß zur Behandlung des Katalysators ein Salz der Zusammensetzung M$^+$X$^-$ verwendet wird, wobei M$^+$ für Li$^+$, Na$^+$, K$^+$, oder NH$_4^+$ und X$^-$ für NO$_3^-$, ClO$_4^-$, Cl$^-$oder H$_2$PO$_4^-$ steht.

4. Verfahren nach Anspruch 1 oder 2, dadurch gekennzeichnet, daß zur Behandlung des Katalysators ein Salz der Zusammensetzung M$_2$SO$_4$ verwendet wird, wobei M$^+$ für Li$^+$, Na$^+$, K$^+$, oder NH$_4^+$ steht.

5. Verfahren nach einem der Ansprüche 1 bis 4, dadurch gekennzeichnet, daß der Katalysator mit einer wäßrigen oder alkoholischen Lösung des Salzes behandelt wird.

6. Verfahren nach Anspruch 5, dadurch gekennzeichnet, daß der Katalysator vor der Verwendung zur Epoxidierung mit der Salzlösung behandelt wird und die zur Behandlung eingesetzte Salzlösung mehrfach zur Behandlung neuer Katalysatormengen wieder verwendet wird.

7. Verfahren nach Anspruch 5, dadurch gekennzeichnet, daß die Behandlung des Katalysators durch Zugabe der Salzlösung zur Epoxidierungsreaktion erfolgt.

8. Verfahren nach einem der Ansprüche 1 bis 4, dadurch gekennzeichnet, daß das zur Behandlung des Katalysators verwendete Salz einem der Einsatzstoffe der Epoxidierungsreaktion zugesetzt wird.

**F i g u r · l**

F i g u r 2

F i g u r  3

Europäisches
Patentamt

**EUROPÄISCHER RECHERCHENBERICHT**

Nummer der Anmeldung

EP 96 10 8370

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,X | JOURNAL OF CATALYSIS, Bd. 140, Nr. 1, März 1993, DULUTH, MN, US, Seiten 71-83, XP000562771 M.G. CLERICI ET AL.: "Epoxidation of lower olefins with hydrogen peroxide and titanium silicalite" * das ganze Dokument, insbesondere Seite 75, Tabelle 4 und linke Spalte, letzter Absatz, sowie Seite 77, rechte Spalte, erster Textabsatz --- | 1-3,5-7 | C07D301/12 |
| X | US-A-5 374 747 (R.J. SAXTON ET AL.) 20.Dezember 1994 * Spalte 3, zeile 53, bis Spalte 4, Zeile 68 * --- | 1-8 | |
| X | US-A-5 412 122 (R.J. SAXTON ET AL.) 2.Mai 1995 * Spalte 3, Zeile 60, bis Spalte 4, sowie Zeile 56, Spalte 8, Zeilen 33-55 * --- | 1-8 | |
| X | EP-A-0 659 685 (ARCO CHEMICAL TECHNOLOGY) 28.Juni 1995 * Seite 6, Zeile 34, bis Seite 7, Zeile 18 * --- | 1-8 | **RECHERCHIERTE SACHGEBIETE (Int.Cl.6)** C07D |
| P,X | EP-A-0 712 852 (ARCO CHEMICAL TECHNOLOGY) 22.Mai 1996 * das ganze Dokument * ----- | 1-8 | |

Der vorliegende Recherchenbericht wurde für alle Patentansprüche erstellt

| Recherchenort | Abschlußdatum der Recherche | Prüfer |
|---|---|---|
| DEN HAAG | 18.Oktober 1996 | Allard, M |